# FASCICULE DE BREVET EUROPEEN

(11) **EP 1 299 369 B1**
(45) Date de publication et mention de la délivrance du brevet: **30.03.2005**
(21) Numéro de dépôt: 01953991.5
(22) Date de dépôt: 26.06.2001
(51) Int. Cl.: C07D 301/12

(54) **PROCEDE DE FABRICATION D'OXIRANNE**
VERFAHREN ZUR HERSTELLUNG VON OXIRANEN
METHOD FOR PRODUCING OXIRANE

(30) Priorité: 28.06.2000 FR 0008354
(43) Date de publication de la demande: 09.04.2003
(73) Titulaire: SOLVAY (Société Anonyme), 1050 Bruxelles (BE)
(72) Inventeur: BALTHASART, Dominique, B-1120 Bruxelles (BE)
(74) Mandataire: Vande Gucht, Anne
(86) Numéro de dépôt international: PCT/EP2001/007271
(87) Numéro de publication internationale: WO 2002/000635

(56) Documents cités:
- WO-A-99/32472

## Description

La présente invention concerne un procédé de fabrication d'oxiranne par réaction entre une oléfine et un composé peroxydé en présence d'un catalyseur et d'un solvant. En particulier, elle concerne la fabrication d'oxyde de propylène (ou d'épichlorhydrine) par époxydation de propylène (ou de chlorure d'allyle) au moyen de peroxyde d'hydrogène en présence d'un catalyseur contenant du TS-1.

Il est connu de fabriquer de l'oxyde de propylène par réaction entre le propylène et un composé peroxydé en présence de TS-1. Par exemple, dans le brevet US 5,849,937 un tel procédé est réalisé dans plusieurs réacteurs disposés en série.

La demanderesse a constaté que ce procédé présente l'inconvénient de conduire à la formation de sous-produits, par exemple lorsque l'on utilise dans ce procédé connu une solution aqueuse de peroxyde d'hydrogène autre de composé peroxydé et du méthanol à titre de solvant. En effet, lorsque l'oxyde de propylène est fabriqué dans ces conditions, des sous-produits sont formés par réaction entre l'oxyde de propylène et l'eau ou le méthanol, et notamment le propylène glycol et des méthoxypropanols de formule CH₃-CHOH-CH₂-OCH₃ et CH₃-CH(OCH₃)-CH₂OH. Lorsque l'épichlorhydrine est fabriquée, des sous-produits sont formés par réaction entre l'épichlorhydrine et l'eau ou le méthanol, et notamment le 1-chloropropanediol et des chlorométhoxypropanols de formule ClCH₂-CHOH-CH₂-OCH₃ et Cl-CH₂-CH(OCH₃)-CH₂OH. La formation de sous-produits réduit la sélectivité du procédé et dès lors son rendement.

La présente invention vise à remédier à cet inconvénient en fournissant un nouveau procédé de sélectivité élevée, tout en gardant une activité (ou vitesse de réaction) élevée.

A cet effet, l'invention concerne un procédé de fabrication d'oxiranne par réaction d'une oléfine avec un composé peroxydé en présence d'un catalyseur et d'un solvant dans au moins deux réacteurs disposés en série dont chacun contient une partie du catalyseur, selon lequel on introduit dans un premier réacteur une première partie de l'oléfine, le solvant et le composé peroxydé, on y effectue une époxydation de la première partie de l'oléfine pour former une première partie de l'oxiranne, on soutire de ce réacteur un milieu comprenant la première partie d'oxiranne formé, le solvant, l'oléfine non convertie et, le cas échéant, le composé peroxydé non consommé, on introduit le milieu dans une colonne à distiller, on recueille en tête de colonne la majeure partie de l'oxiranne formé et de l'oléfine non convertie, on recueille en pied de colonne le milieu appauvri en oxiranne contenant, le cas échéant, le composé peroxydé non consommé, on introduit dans un réacteur ultérieur le milieu appauvri en oxiranne et une autre partie de l'oléfine et éventuellement une autre partie du composé peroxydé, on y effectue une époxydation de l'autre partie de l'oléfine pour former une autre partie de l'oxiranne, et on recueille l'autre partie de l'oxiranne ainsi formé.

Une des caractéristiques essentielles de la présente invention réside dans la distillation intermédiaire effectuée entre les deux réactions d'époxydation. Ceci permet en effet de minimaliser la formation de sous-produits.

La distillation a pour fonction d'éliminer le plus vite possible l'oxiranne dès sa formation dans le milieu réactionnel pour éviter qu'il entre en contact avec les autres constituants du milieu réactionnel et pour éviter que des sous-produits soient ainsi formés. Cette séparation est effectuée dans une colonne à distiller séparée et distincte du réacteur d'époxydation. Puisque le catalyseur ne quitte pas le réacteur d'époxydation, la distillation est donc effectuée en l'absence du catalyseur d'époxydation afin d'éviter le contact entre l'oxiranne formé et le catalyseur d'époxydation car celui-ci favorise la formation des sous-produits.

La distillation permet de séparer la majeure partie de l'oxiranne formé du milieu réactionnel d'époxydation. Cette majeure partie est en général supérieure
ou égale à 80 % de la quantité d'oxiranne formé dans le premier réacteur. Elle est le plus souvent supérieure ou égale à 90 %. Habituellement, elle est inférieure ou égale à 99 %. En particulier inférieure ou égale à 95 %.

Les conditions dans lesquelles sont réalisées la distillation dépendent de la nature de l'oxiranne (et notamment de sa température d'ébullition), de sa concentration dans le milieu introduit dans la colonne à distiller, de la nature des autres constituants du milieu (l'oléfine non convertie et le solvant), de leur température d'ébullition et du rendement souhaité de la distillation.

La distillation est généralement réalisée à une température supérieure ou égale à 10 °C, de préférence supérieure ou égale à 35 °C, les valeurs supérieures
ou égales à 45 °C étant recommandées. La température est habituellement inférieure ou égale à 125 °C, le plus souvent inférieure ou égale à 100 °C, les valeurs inférieures ou égales à 90 °C étant préférées.

La distillation est couramment effectuée à une pression supérieure ou égale à 0,1 bar, de préférence supérieure ou égale à 0,5 bar, les valeurs supérieures ou égales à 1 bar étant les plus courantes. La pression est en général inférieure ou égale à 10 bar, en particulier inférieure ou égale à 5 bar, les valeurs inférieures ou égales à 2 bar étant tout particulièrement recommandées. Dans la présente description, toute référence à la pression de la distillation correspond à la pression absolue mesurée en tête de la colonne de distillation.

La colonne de distillation utilisable dans le procédé selon l'invention est connue en elle-même. On peut utiliser, par exemple, une colonne à plateaux conventionnels ou une colonne à plateaux de type "dual-flow" ou encore une colonne à empilage en vrac ou structuré.

Le nombre de plateaux théoriques dans la colonne à distiller est généralement supérieur ou égal à 20, plus spécialement supérieur ou égal à 30. Un nombre inférieur ou égal à 80 donne de bons résultats. Un nombre inférieur ou égal à 60 est recommandé.

Le taux de reflux molaire (qui correspond au débit molaire de liquide renvoyé en tête de colonne ramené à l'ensemble du distillat - vapeur plus liquide - prélevé en tête de colonne) dans la colonne à distiller est habituellement supérieur ou égal à 0,5, de préférence supérieur ou égal à 0,8. Ce taux est couramment inférieur ou égal à 5, le plus souvent inférieur ou égal à 2,5.

Dans le procédé selon l'invention on met en oeuvre une installation comprenant au moins deux réacteurs d'époxydation disposés en série et reliés entre eux. Chaque réacteur est alimenté en oléfine. Le composé peroxydé et le solvant sont introduits dans le premier réacteur. Du composé peroxydé frais peut également être introduit dans un ou plusieurs des réacteurs ultérieurs. Chaque réacteur contient une partie du catalyseur qui ne quitte pas ce réacteur. Lorsque le catalyseur est présent sous la forme d'un lit fixe, il n'est en général pas nécessaire de prendre des précautions pour maintenir le catalyseur dans le réacteur. En variante, le catalyseur peut être présent sous la forme de particules dont une partie au moins est à l'état fluidisé par un courant liquide ou par agitation mécanique ou par un gaz. Lorsqu'on utilise un courant liquide, il est recommandé de prévoir une zone de dégagement surmontant le lit fluide pour arrêter les particules de catalyseur qui sont en mouvement et/ou de prévoir un filtre à la sortie du réacteur.

L'installation peut évidemment comprendre plus de deux réacteurs connectés en série. Dans ce cas, le premier réacteur de la série est alimenté avec l'oléfine, le composé peroxydé, le solvant (et éventuellement une fraction du milieu obtenu en pied de la colonne à distiller correspondant à ce réacteur) et chaque réacteur ultérieur est alimenté avec l'oléfine, le solde du milieu provenant du réacteur précédent de la série et éventuellement du composé peroxydé frais. On utilise de préférence 3 réacteurs en série.

Dans le procédé selon l'invention, on utilise de préférence des réacteurs de dimension identique. Ceci permet de permuter la fonction des réacteurs lorsque le catalyseur désactivé d'un réacteur est remplacé par du catalyseur frais ou régénéré sans perturber le fonctionnement de l'installation (fonctionnement dit "en carrousel").

Dans une première forme de réalisation du procédé selon l'invention, non seulement le premier réacteur de la série mais chaque réacteur ultérieur de la série est suivi d'une colonne de distillation, appelée dans ce qui suit "distillation ultérieure". Cette distillation ultérieure a la même fonction que la distillation intermédiaire effectuée entre la première et deuxième époxydation. Les conditions des distillations ultérieures sont similaires aux conditions décrites plus haut pour la distillation intermédiaire. Dans cette forme de réalisation, on introduit le milieu sortant de chaque réacteur ultérieur et contenant l'autre partie de l'oxiranne, le solvant, l'oléfine non convertie et, le cas échéant, le composé peroxyde non consommé dans une colonne à distiller ultérieure, on recueille en tête de colonne l'autre partie de l'oxiranne et l'oléfine non convertie, et en pied de colonne le solvant et éventuellement le composé peroxydé non consommé: Dans un procédé continu, il peut être avantageux de recycler le milieu recueilli en pied de la dernière colonne à distiller (et contenant le solvant et éventuellement le composé peroxydé non consommé) dans le premier réacteur. Par exemple, lorsque le procédé est réalisé dans deux réacteurs en série, le milieu recueilli en pied de la deuxième colonne à distiller peut être recyclé dans le premier réacteur.

Dans une deuxième forme de réalisation du procédé selon l'invention, on introduit le mélange d'oxiranne et d'oléfine non convertie recueilli en tête de colonne à distiller (première et/ou ultérieure) dans un récipient dans lequel l'oxiranne est séparé de l'oléfine non convertie. Cette oléfine non convertie peut alors être recyclée dans un des réacteurs, de préférence dans le premier réacteur. Le récipient peut contenir un condenseur ou un liquide absorbant ou un solide absorbant ou encore une membrane perméosélective. Les condenseurs conviennent bien.

Une troisième forme de réalisation du procédé selon l'invention consiste à utiliser le catalyseur sous forme de particules dont une partie au moins se trouve à l'état fluidisé, comme décrit dans la demande de brevet de la demanderesse déposée le même jour que la présente demande de brevet et intitulée "Procédé de fabrication d'oxiranne en présence d'un catalyseur sous forme de particules" (dont le contenu est incorporé par référence). Dans ce cas, il est recommandé de prévoir un filtre à travers duquel passe le milieu sortant du réacteur (premier et/ou ultérieur) avant d'être introduit dans la colonne à distiller (première et/ou ultérieure). Cette forme de réalisation permet d'obtenir une dispersion homogène du catalyseur dans le milieu réactionnel d'époxydation, un bon échange thermique et donc un contrôle aisé de la température de réaction.

Dans une quatrième forme de réalisation du procédé selon l'invention, on soumet le milieu entrant dans la colonne à distiller (première et/ou ultérieure) d'abord à une détente avant d'être introduit dans cette colonne à distiller. Cette forme convient particulièrement bien lorsque l'époxydation est réalisée sous pression ou en présence d'un composé gazeux. Ce composé gazeux peut être l'oléfine même (par exemple le propylène) ou un gaz inerte que l'on introduit dans le milieu réactionnel d'époxydation pour permettre d'entraîner l'oxiranne et . de le sortir du réacteur, comme décrit dans la demande de brevet WO 99/48883 de la demanderesse.

Dans une cinquième forme de réalisation du procédé selon l'invention, l'entièreté du composé peroxyde est introduite dans le premier réacteur, comme décrit dans la demande de brevet de la demanderesse déposée le même jour et intitulée "Procédé de fabrication d'oxiranne au moyen d'un composé peroxydé" (dont le contenu est incorporé par référence). Le ou les réacteurs ultérieurs ne sont donc pas alimentés en composé peroxydé frais mais seulement avec le composé peroxydé qui est présent dans le milieu provenant du réacteur précédent et qui n'a pas été consommé dans ce réacteur précédent. En général, on introduit également de l'eau avec le composé peroxydé dans le premier réacteur. Le fait de ne pas ajouter du composé peroxydé dans le(s) réacteur(s) ultérieurs) permet de consommer 100 % de la quantité totale de composé peroxydé mise en oeuvre sans pour autant diminuer la vitesse de réaction par rapport à un procédé utilisant la même quantité totale de composé peroxydé mais dans lequel chaque réacteur est alimenté en composé peroxydé frais.

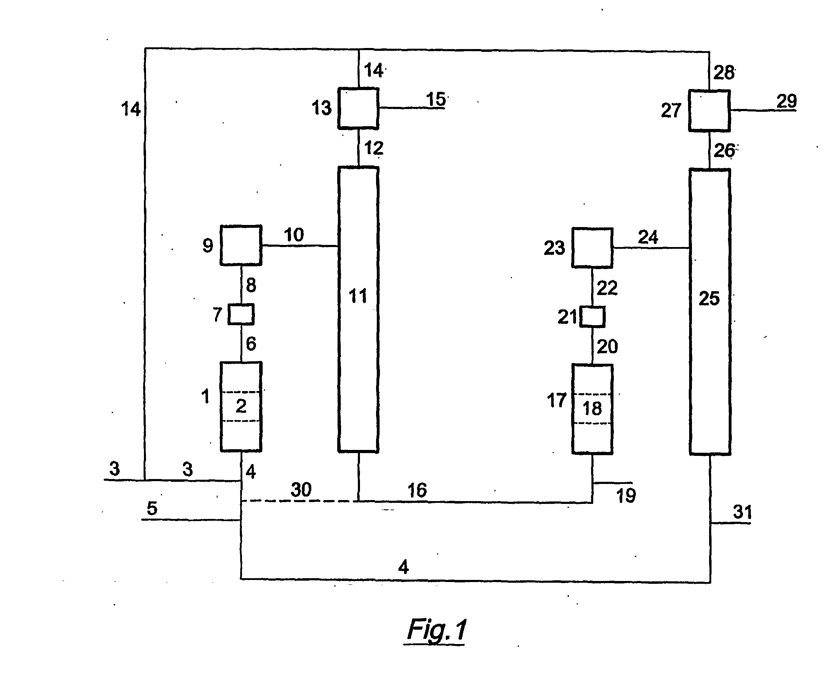

Une forme de réalisation préférée du procédé selon l'invention est schématisée dans la figure 1. Dans cette forme préférée, le premier réacteur 1 contient une partie du catalyseur, de préférence en lit fluide 2. Le réacteur 1 est alimenté en une première partie de l'oléfine par le conduit 3 et ensuite par le conduit 4, en composé peroxydé par le conduit 5 en ensuite par le conduit 4, et en solvant par le conduit 4 provenant d'une autre partie de l'installation qui est décrite plus loin. Dans le premier réacteur, la première partie de l'oléfine réagit avec le composé peroxydé en présence du catalyseur pour former une première partie de l'oxiranne. Le milieu sortant du réacteur 1 via le conduit 6 contient le solvant, la première partie de l'oxiranne, le composé peroxydé non consommé et l'oléfine non convertie. Ce milieu passe à travers un filtre 7, et est envoyé via le conduit 8 dans le récipient 9 où il est soumis à une détente. Le milieu est ensuite transporté via le conduit 10 dans une colonne à distiller 11. En tête de cette colonne à distiller 11, on récupère un mélange d'oxiranne et d'oléfine non convertie. Ce mélange est envoyé via le conduit 12 dans un condenseur 13 qui sépare l'oxiranne de l'oléfine non convertie. L'oléfine non convertie est recyclée dans le réacteur 1 via les conduits 14, 3 et 4. La première partie d'oxiranne est recueillie comme produit fini via le conduit 15. En pied de la colonne à distiller 11, on recueille un milieu contenant le solvant, le composé peroxydé non consommé dans le réacteur 1 et éventuellement une partie de l'oléfine non convertie. Ce milieu, dont une partie peut éventuellement être recyclée dans le réacteur 1 via le conduit 30, est transporté via le conduit 16 dans un deuxième réacteur 17 contenant une autre partie du catalyseur, de préférence à l'état d'un lit fluide 18. Le deuxième réacteur 17 est alimenté en une deuxième partie de l'oléfine via le conduit 19. Dans le deuxième réacteur 17, la deuxième partie de l'oléfine réagit avec le composé peroxydé non consommé provenant du premier réacteur en présence du catalyseur 18 pour former une deuxième partie de l'oxiranne. Les conditions dans le deuxième réacteur 17 sont de préférence telles que la totalité du composé peroxydé provenant du premier réacteur soit consommée. Le milieu sortant du réacteur 17 via le conduit 20 contient alors le solvant, la deuxième partie de l'oxiranne et l'oléfine non convertie. Ce milieu passe à travers un filtre 21, et est envoyé via le conduit 22 dans le récipient 23 où il est soumis à une détente. Le milieu est ensuite transporté via le conduit 24 dans une deuxième colonne à distiller 25. En tête de cette colonne à distiller 25, on récupère un mélange de la deuxième partie d'oxiranne et d'oléfine non convertie. Ce mélange est envoyé via le conduit 26 dans un condenseur 27 qui sépare l'oxiranne de l'oléfine non convertie. L'oléfine non convertie est recyclée dans le réacteur 1 via les conduits 28, 14, 3 et 4. La deuxième partie d'oxiranne est recueillie comme produit fini via le conduit 29. En pied de la colonne à distiller 25, on recueille le solvant qui est recyclé via le conduit 4 dans le premier réacteur 1, et un effluent aqueux qui est évacué via le conduit 31.

Le catalyseur utilisé dans.le procédé selon l'invention contient généralement une zéolite comme élément actif, et de manière préférée, une zéolite au titane. Par zéolite au titane, on entend désigner un solide contenant de la silice qui présente une structure cristalline microporeuse de type zéolite et dans laquelle plusieurs atomes de silicium sont remplacés par des atomes de titane. La zéolite au titane présente avantageusement une structure cristalline de type ZSM-5, ZSM-11, ZSM-12, MCM-41, ZSM-48. Elle peut aussi présenter une structure cristalline de type zéolite bêta, de préférence exempte d'aluminium. Les zéolites présentant une bande d'absorption infrarouge à environ 950-960 cm-1 conviennent bien. Les zéolites au titane de type silicalite sont préférées. Celles répondant à la formule xTiO₂(1-x)SiO₂ dans laquelle x est de 0,0001 à 0,5 de préférence de 0,001 à 0,05, sont performantes. Des matériaux de ce type, connus sous le nom de TS-1, présentent une structure zéolitique cristalline microporeuse analogue à celle de la zéolite ZSM-5.

Le catalyseur utilisé dans le procédé selon l'invention se présente avantageusement sous la forme de particules obtenues par extrusion comme décrit dans la demande de brevet WO 99/28029 de la demanderesse, ou par un procédé en spray comme décrit dans la demande de brevet WO 99/24164 de la demanderesse. Le contenu de ces deux demandes de brevet est incorporé par référence dans celle-ci.

Le solvant mis en oeuvre dans le procédé selon l'invention peut être choisi parmi les alcools aliphatiques saturés, linéaires ou branchés. Le solvant alcoolique contient généralement jusqu'à 10 atomes de carbone, de préférence de 1 à 6 atomes de carbone. On peut citer à titre d'exemples le méthanol et l'éthanol. Le méthanol est préféré.

La quantité de solvant mise en oeuvre dans le premier réacteur est généralement d'au moins 25 % en poids du milieu réactionnel liquide présent dans le premier réacteur, en particulier d'au moins 40 % en poids, par exemple d'au moins 50 % en poids. Cette quantité ne dépasse habituellement pas 99 % en poids, en particulier pas 95 % en poids.

Le rapport molaire entre les quantités d'oléfine et de composé peroxydé engagées dans le procédé selon l'invention est généralement d'au moins 0.1, en particulier d'au moins 0,2, et de préférence d'au moins 0,5. Ce rapport molaire est le plus souvent d'au plus 100, en particulier d'au plus 50 et de préférence d'au plus 25.

Le procédé selon l'invention peut être continu ou discontinu.

Dans le procédé selon l'invention, lorsqu'il est réalisé en continu, le composé peroxydé est généralement mis en oeuvre dans le premier réacteur en une quantité d'au moins 0,005 mole par heure et par gramme de catalyseur présent dans le premier réacteur, en particulier, d'au moins 0,01 mole. La quantité de composé peroxydé est habituellement inférieure ou égale à 25 moles et, en particulier, inférieure ou égale à 10 moles. Une préférence est montrée pour une quantité de composé peroxyde supérieure ou égale à 0,03 mole et inférieure ou égale à 2,5 mole.

Dans le procédé selon l'invention le composé peroxydé est avantageusement mis en oeuvre sous forme d'une solution aqueuse. En général, la solution aqueuse contient au moins 2 % en poids de composé peroxydé, en particulier au moins 5 % en poids. Elle contient le plus souvent au maximum 90 % en poids de composé peroxydé, en particulier 70 % en poids.

La température de la réaction entre l'oléfine et le composé peroxydé peut varier de 10 à 125 °C. Dans une variante avantageuse telle que décrite dans la demande de brevet EP99/08703 de la demanderesse, elle est supérieure à 35 °C pour remédier à la désactivation progressive du catalyseur. La température peut être supérieure ou égale à 40 °C et de préférence supérieure ou égale à 45 °C. Une température supérieure ou égale à 50 °C est tout particulièrement préférée. La température de réaction est de préférence inférieure à 100 °C.

Dans le procédé selon l'invention, la réaction entre l'oléfine et le composé peroxydé peut avoir lieu à pression atmosphérique. Elle peut également se dérouler sous pression. Généralement, cette pression n'excède pas 40 bar. Une pression de 20 bar convient bien en pratique.

Les composés peroxydés qui peuvent être utilisés dans le procédé selon l'invention sont les composés peroxydés contenant une ou plusieurs fonctions peroxyde (-OOH) qui peuvent libérer de l'oxygène actif et capables d'effectuer une époxydation. Le peroxyde d'hydrogène et les composés peroxydés qui peuvent produire du peroxyde d'hydrogène dans les conditions de la réaction d'époxydation conviennent bien. Le peroxyde d'hydrogène est préféré.

Lorsqu'on utilise du peroxyde d'hydrogène, il peut être intéressant de mettre en oeuvre dans le procédé selon l'invention une solution aqueuse de peroxyde d'hydrogène à l'état brut, c'est-à-dire non épurée. Par exemple, on peut mettre en oeuvre une solution obtenue par simple extraction avec de l'eau substantiellement pure du mélange issu de l'oxydation d'au moins une alkylanthrahydroquinone (procédé appelé "procédé AO auto-oxydation") sans traitement ultérieur de lavage et/ou de purification. Ces solutions brutes de peroxyde d'hydrogène contiennent généralement de 0,001 à 10 g/l d'impuretés organiques exprimées en COT (Carbone Organique Total). Elles contiennent habituellement des cations métalliques (tels que des métaux alcalins ou alcalino-terreux, comme le sodium) et des anions (tels que les phosphates, nitrates) en des teneurs de 0,01 à 10 g/l.

Dans une variante du procédé, on peut mettre en oeuvre une solution de peroxyde d'hydrogène produite par synthèse directe à partir d'oxygène et d'hydrogène en présence de méthanol.

L'oxiranne qui peut être préparé par le procédé selon l'invention est un composé organique comprenant un groupement répondant à la formule générale :

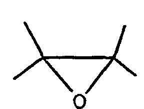

L'oxiranne contient généralement de 2 à 10 atomes de carbone, de préférence de 3 à 6 atomes de carbone. Les oxirannes qui peuvent être préparés de manière avantageuse par le procédé selon l'invention sont le 1,2-époxypropane et le 1,2-époxy-3-chloropropane. L'oxiranné préféré est le 1,2-époxypropane.

Les oléfines qui conviennent bien dans le procédé selon l'invention contiennent généralement de 2 à 10 atomes de carbone et de manière préférée, 3 à 6 atomes de carbone. Le propylène, le butylène et le chlorure d'allyle conviennent bien. Le propylène et le chlorure d'allyle sont préférés. Le propylène est particulièrement préféré.

Dans le procédé selon l'invention il peut s'avérer intéressant de contrôler le pH de la phase liquide. Par exemple, il peut être intéressant de maintenir le pH de la phase liquide lors de la réaction entre l'oléfine et le composé peroxydé à une valeur de 4,8 à 6,5, par exemple par addition d'une base (hydroxyde de sodium) au milieu d'époxydation, comme recommandé dans la demande de brevet WO 99/48882 de la demanderesse (dont le contenu est incorporé par référence dans la présente demande de brevet). Cette base peut être introduite dans un seul réacteur (par exemple, le premier réacteur) ou dans plusieurs réacteurs. Elle est de préférence introduite dans chaque réacteur.

La réaction entre l'oléfine et le composé peroxydé peut s'effectuer en présence d'un sel tel que le chlorure de sodium, comme décrit dans la demande de brevet WO EP99/08703 de la demanderesse (dont le contenu est incorporé par référence dans la présente demande de brevet). Ce sel peut être introduit dans un seul réacteur (par exemple, le premier réacteur) ou dans plusieurs réacteurs. Il est de préférence introduit dans chaque réacteur.

Il peut être avantageux d'introduire l'oléfine à l'état dilué dans un ou plusieurs alcanes. Par exemple, on peut introduire dans les réacteurs d'époxydation un fluide contenant l'oléfine et également au moins 10 % (en particulier 20 %, par exemple au moins 30 %) en volume d'un ou plusieurs alcanes. Par exemple, dans le cas du propylène, celui-ci peut être mélangé avec au moins 10 % en volume de propane lorsqu'on introduit dans le réacteur le propylène non converti recyclé. Il peut également s'agir d'une source de propylène incomplètement épurée en propane.

Les exemples qui suivent sont destinés à illustrer la présente invention sans toutefois en limiter la portée.

Les exemples 1 et 2 ont été calculés au moyen du logiciel ASPEN PLUS® de la société ASPEN TECHNOLOGY INC. à l'aide des paramètres cinétiques de la réaction déterminés sur base des essais expérimentaux décrits et des équilibres liquide-vapeur disponibles dans la littérature.

### Exemple comparatif 1

Dans cet exemple, la synthèse de l'oxyde de propylène est effectuée dans 2 réacteurs en série sans séparation intermédiaire de l'oxyde de propylène formé au premier réacteur.

326.5 kmol/h de peroxyde d'hydrogène accompagné de 1100 kmol/h d'eau sont mélangés à 1500 kmol/h de méthanol et à 250 kmol/h de propylène sous une pression suffisante pour solubiliser tout le propylène à la température .de réaction. Le mélange réactionnel est introduit en continu à 70°C dans un réacteur méthodique contenant 600 kg de catalyseur.

L'effluent du réacteur est mélangé à 200 kmol/h de propylène à une pression suffisante pour solubiliser l'entièreté du propylène à la température de réaction et introduit en continu à 70°C dans un second réacteur méthodique contenant 600 kg de catalyseur.

L'effluent du second réacteur contient 2.3 kmol/h de peroxyde d'hydrogène non converti, 243.9 kmol/h d'oxyde de propylène et 77.4 kmol/h de sous-produits (methoxypropanol et propanediol principalement) ; le rendement en C3 atteint 74.7% pour un taux de conversion du peroxyde d'hydrogène de 99.3%.

### Exemple 2 (conforme à l'invention)

Dans cet exemple, la synthèse de l'oxyde de propylène est effectuée dans 2 réacteurs en série avec séparation intermédiaire de l'oxyde de propylène formé au premier réacteur.

326.5 kmol/h de peroxyde d'hydrogène accompagné de 1100 kmol/h d'eau sont mélangés à 1500 kmol/h de méthanol et à 250 kmol/h de propylène sous une pression suffisante pour solubiliser tout le propylène à la température de réaction. Le mélange réactionnel est introduit en continu à 70°C dans un réacteur méthodique contenant 600 kg de catalyseur.

L'effluent du réacteur est dirigé vers une colonne de rectification contenant 50 plateaux théoriques (y compris condenseur et bouilleur) ; l'alimentation est effectuée au niveau du 10^{ème} plateau théorique (compté à partir du condenseur) ; la colonne est opérée à 1.1 bar absolu (pression de tête de colonne) ; la température de tête de colonne est maintenue à 40°C (distillat partiellement vaporisé) ; le taux de reflux molaire est fixé à 1 ; le débit de distillat est ajusté de façon à récupérer en tête de colonne 95% de l'oxyde de propylène présent dans l'alimentation de la colonne.

Le mélange soutiré en pied de colonne, appauvri en oxyde de propylène, est mélangé à 200 kmol/h de propylène à une pression suffisante pour solubiliser l'entièreté du propylène à la température de réaction et introduit en continu à 70°C dans un second réacteur méthodique contenant 600 kg de catalyseur.

L'effluent du second réacteur contient 0.5 kmol/h de peroxyde d'hydrogène non converti, 85.9 kmol/h d'oxyde de propylène et 68.5 kmol/h de sous-produits (methoxypropanol et propanediol principalement) ; le distillat de la colonne contient 168.8 kmollh d'oxyde de propylène ; le rendement en C3 atteint 78.0% pour un taux de conversion du peroxyde d'hydrogène de 99.9%.

## Revendications

1. Procédé de fabrication d'oxiranne par réaction d'une oléfine avec un composé peroxydé en présence d'un catalyseur et d'un solvant dans au moins deux réacteurs disposés en série, dont chacun contient une partie du catalyseur, selon lequel on introduit dans un premier réacteur une première partie de l'oléfine, le solvant et le composé peroxydé, on y effectue une époxydation de la première partie de l'oléfine pour former une première partie de l'oxiranne, on soutire de ce réacteur un milieu comprenant la première partie d'oxiranne formé, le solvant, l'oléfine non convertie et, le cas échéant, le composé peroxydé non consommé, on introduit le milieu dans une colonne à distiller, on recueille en tête de colonne la majeure partie de l'oxiranne formé et de l'oléfine non convertie, on recueille en pied de colonne le milieu appauvri en oxiranne contenant, le cas échéant, le composé peroxydé non consommé, on introduit dans un réacteur ultérieur le milieu appauvri en oxiranne et une autre partie de l'oléfine et éventuellement une autre partie du composé peroxydé, on y effectue une époxydation de l'autre partie de l'oléfine pour former une autre partie de l'oxiranne, et on recueille l'autre partie de l'oxiranne ainsi formé.

2. Procédé selon la revendication 1, dans lequel on introduit le milieu sortant du réacteur ultérieur et contenant l'autre partie de l'oxiranne, le solvant, l'oléfine non convertie et éventuellement le composé peroxydé non consommé dans une colonne à distiller ultérieure, on recueille en tête de cette colonne la majeure partie de l'autre partie de l'oxiranne et de l'oléfine non convertie, et en pied de cette colonne le solvant et éventuellement le composé peroxydé non consommé.

3. Procédé selon la revendication 2, dans lequel le procédé est réalisé dans deux réacteurs disposés en série et dans lequel le milieu recueilli en pied de la deuxième colonne à distiller est recyclé dans le premier réacteur.

4. Procédé selon l'une quelconque des revendications 1 à 3, dans lequel le mélange d'oxiranne et d'oléfine non convertie recueilli en tête de colonne à distiller (première et/ou ultérieure) est introduit dans un condenseur dans lequel l'oxiranne est séparé de l'oléfine non convertie que l'on recycle dans un des réacteurs.

5. Procédé selon l'une quelconque des revendications 1 à 4, dans lequel tous les réacteurs sont de dimension identique.

6. Procédé selon l'une quelconque des revendications 1 à 5, dans lequel le catalyseur est présent dans chaque réacteur sous forme de particules dont une partie au moins se trouve à l'état fluidisé.

7. Procédé selon la revendication 6, dans lequel le milieu sortant du réacteur (premier et/ou ultérieur) passe à travers un filtre avant d'être introduit dans la colonne à distiller (première et/ou ultérieure).

8. Procédé selon l'une quelconque des revendications 1 à 7, dans lequel le milieu entrant dans la colonne à distiller (première et/ou ultérieure) est d'abord soumis à une détente avant d'être introduit dans cette colonne à distiller.

9. Procédé selon l'une quelconque des revendications 1 à 8, dans lequel l'entièreté du composé peroxydé est introduite dans le premier réacteur.

10. Procédé selon l'une quelconque des revendications 1 à 9, dans lequel l'oxiranne est l'oxyde de propylène ou l'épichlorhydrine, l'oléfine est le propylène ou le chlorure d'allyle, le composé peroxyde est le peroxyde d'hydrogène, le solvant est le méthanol et le catalyseur contient du TS-1.

11. Procédé selon la revendication 10, dans lequel du peroxyde d'hydrogène, de l'eau, du méthanol et du propylène sous pression sont introduits en continu dans le premier réacteur contenant du catalyseur, l'effluent du premier réacteur est dirigé vers la colonne à distiller à la tête de laquelle de l'oxyde de propylène est récupéré, le mélange soutiré en pied de la colonne, appauvri en oxyde de propylène, est mélangé uniquement à du propylène sous pression et introduit en continu dans le second réacteur contenant du catalyseur, et l'effluent du second réacteur est récupéré.

12. Procédé selon l'une quelconque des revendications 1 à 11, dans lequel 3 réacteurs en série sont utilisés.

## Patentansprüche

1. Verfahren zur Herstellung von Oxiran durch Umsetzung eines Olefins mit einer Peroxidverbindung in Gegenwart eines Katalysators und eines Lösungsmittels in wenigstens zwei, in Reihe angeordneten Reaktoren, von denen jeder einen Teil des Katalysator enthält, wonach in einen ersten Reaktor ein erster Teil des Olefins, das Lösungsmittel und die Peroxidverbindung eingeführt werden, darin eine Epoxidation des ersten Teils des Olefins vorgenommen wird, um einen ersten Teil des Oxirans auszubilden, aus diesem Reaktor ein Milieu abgezogen wird, das den ersten Teil des gebildeten Oxirans, das Lösungsmittel, das nicht umgewandelte Olefin und gegebenenfalls die nicht verbrauchte Peroxidverbindung umfaßt, das Milieu in eine Destillationskolonne eingeführt wird, am Kopf der Kolonne der Hauptteil des gebildeten Oxirans und des nicht umgewandelten Olefms gewonnen wird, am Kolonnensumpf das an Oxiran verarmte Milieu gewonnen wird, das gegebenenfalls die nicht verbrauchte Peroxidverbindung enthält, in einen nachfolgenden Reaktor das an Oxiran verarmte Milieu und ein weiterer Teil des Olefins und gegebenenfalls ein weiterer Teil der Peroxidverbindung eingeführt werden, darin eine Epoxidation des anderen Teils des Olefins vorgenommen wird, um einen anderen Teil des Oxirans auszubilden, und der andere Teil des solcherart gebildeten Oxirans gewonnen wird.

2. Verfahren nach Anspruch 1, worin das aus dem nachfolgenden Reaktor austretende Milieu mit einem Gehalt an dem anderen Teil des Oxirans, Lösungsmittel, nicht umgewandeltem Olefin und gegebenenfalls nicht verbrauchter Peroxidverbindung in eine nachfolgende Destillationskolonne eingeführt wird, am Kopf dieser Kolonne der Hauptteil des anderen Teils des Oxirans und des nicht umgewandelten Olefins gewonnen wird und am Sumpf dieser Kolonne das Lösungsmittel und gegebenenfalls die nicht verbrauchte Peroxidverbindung gewonnen werden.

3. Verfahren nach Anspruch 2, worin das Verfahren in zwei, in Reihe angeordneten Reaktoren ausgeführt wird und worin das am Sumpf der zweiten Destillationskolonne gewonnene Milieu in den ersten Reaktor recycliert wird.

4. Verfahren nach einem der Ansprüche 1 bis 3, worin das am Kopf der Destillationskolonne (erste und/oder nachfolgende) gewonnene Gemisch aus Oxiran und nicht umgewandeltem Olefin in einen Kondensator eingeführt wird, worin das Oxiran vom nicht umgewandeltem Olefin abgetrennt wird, das zu einem der Reaktoren recycliert wird.

5. Verfahren nach einem der Ansprüche 1 bis 4, worin sämtliche Reaktoren identische Abmessungen aufweisen.

6. Verfahren nach einem der Ansprüche 1 bis 5, worin der Katalysator in jedem Reaktor in Form von Teilchen vorliegt, von denen sich wenigstens ein Teil im fluidisierten Zustand befindet.

7. Verfahren nach Anspruch 6, worin das aus dem Reaktor (erster und/oder nachfolgender) austretende Milieu ein Filter passiert, bevor es in die Destillationskolonne (erste und/oder nachfolgende) eingeführt wird.

8. Verfahren nach einem der Ansprüche 1 bis 7, worin das in die Destillationskolonne (erste und/oder nachfolgende) eintretende Milieu zunächst einer Entspannung unterzogen wird, bevor es in diese Destillationskolonne eingerührt wird.

9. Verfahren nach einem der Ansprüche 1 bis 8, worin die gesamte Peroxidverbindung in den ersten Reaktor eingebracht wird.

10. Verfahren nach einem der Ansprüche 1 bis 9, worin das Oxiran das Propylenoxid oder Epichlorhydrin ist, das Olefin Propylen oder Allylchlorid ist, die Peroxidverbindung Wasserstoffperoxid ist, das Lösungsmittel Methanol ist und der Katalysator TS-1 enthält.

11. Verfahren nach Anspruch 10, worin Wasserstoffperoxyd, Wasser, Methanol und Propylen unter Druck in den ersten Reaktor welcher Katalysator enthält kontinuierlich eingebracht werden, das aus dem ersten Reaktor abgezogene Milieu der Destillationskolonne zugeführt wird, an deren Kopf Propylenoxid gewonnen wird, das am Kolonnensumpf abgezogene an Propylenoxid verarmte Milieu unter Druck ausschließlich mit Propylen gemischt und kontinuierlich in den zweiten, Katalysator enthaltenden Reaktor eingeführt wird, und das aus dem zweiten Reaktor austretende Milieu gewonnen wird.

12. Verfahren nach einem der Ansprüche 1 bis 11, in dem 3 Reaktoren in Reihe verwendet werden.

## Claims

1. Process for manufacturing oxirane by reacting of an olefin with a peroxide compound in the presence of a catalyst and a solvent in at least two reactors arranged in series, each of which contains a portion of the catalyst, according to which a first portion of the olefin, the solvent and the peroxide compound are introduced into a first reactor, an epoxidation of the first portion of the olefin is carried out therein in order to form a first portion of the oxirane, a medium comprising the first portion of oxirane formed, the solvent, the unconverted olefin and, where appropriate, the unconsumed peroxide compound is removed from this reactor and introduced into a distillation column, the majority of the oxirane formed and of the unconverted olefin is collected at the top of the column, the medium depleted in oxirane and containing, where appropriate, the unconsumed peroxide compound is collected at the bottom of the column, the medium depleted in oxirane and another portion of the olefin and optionally another portion of the peroxide compound are introduced into a subsequent reactor, an epoxidation of the other portion of the olefin is carried out therein in order to form another portion of the oxirane, and the other portion of the oxirane thus formed is collected.

2. Process according to Claim 1, in which the medium leaving the subsequent reactor and containing the other portion of the oxirane, the solvent, the unconverted olefin and possibly the unconsumed peroxide compound is introduced into a subsequent distillation column, the majority of the other portion of the oxirane and of the unconverted olefin are collected at the top of this column and the solvent and possibly the unconsumed peroxide compound are collected at the bottom of this column.

3. Process according to Claim 2, in which the process is carried out in two reactors arranged in series and in which the medium collected at the bottom of the second distillation column is recycled into the first reactor.

4. Process according to any one of Claims 1 to 3, in which the mixture of oxirane and of unconverted olefin collected at the top of the distillation column (first and/or subsequent column) is introduced into a condenser in which the oxirane is separated from the unconverted olefin which is recycled into one of the reactors.

5. Process according to any one of Claims 1 to 4, in which all the reactors are of identical size.

6. Process according to any one of Claims 1 to 5, in which the catalyst is present in each reactor in the form of particles, at least a portion of which is in fluidized form.

7. Process according to Claim 6, in which the medium leaving the reactor (first and/or subsequent reactor) passes through a filter before being introduced into the distillation column (first and/or subsequent column).

8. Process according to any one of Claims 1 to 7, in which the medium entering the distillation column (first and/or subsequent column) is first subjected to a depressurization before being introduced into this distillation column.

9. Process according to any one of Claims 1 to 8, in which all of the peroxide compound is introduced into the first reactor.

10. Process according to any one of Claims 1 to 9, in which the oxirane is propylene oxide or epichlorohydrin, the olefin is propylene or allyl chloride, the peroxide compound is hydrogen peroxide, the solvent is methanol and the catalyst contains TS-1.

11. Process according to Claim 10, in which hydrogen peroxide, water, methanol and propylene under pressure are introduced continuously into the first reactor containing catalyst, the effluent from the first reactor is directed towards the distillation column at the top of which propylene oxide is recovered, the mixture drawn off at the bottom of the column, depleted in propylene oxide, is mixed solely with propylene under pressure and introduced continuously into the subsequent reactor containing catalyst, and the effluent from the subsequent reactor is collected.

12. Process according to any one of Claims 1 to 11, in which 3 reactors in series are used.
